# EUROPEAN PATENT APPLICATION

(11) **EP 4 617 663 A1**
(43) Date of publication of application: **17.09.2025**
(21) Application number: 23889111.3
(22) Date of filing: 07.11.2023
(51) Int. Cl.: G01N 33/574, G01N 33/543

(54) **KIT FOR DIAGNOSING CANCER COMPRISING PROTEIN BIOMARKER IN BLOOD**

(30) Priority: 10.11.2022 KR 20220149875
(71) Applicant: Eulji University Industry Academy Cooperation Foundation, Seongnam-si, Gyeonggi-do 13135 (KR)
(72) Inventor: CHO, Il-Hoon, Seongnam-si Gyeonggi-do 13566 (KR); HWANG, Young Sun, Seoul 04086 (KR)
(74) Representative: Dehns
(86) International application number: PCT/KR2023/017778
(87) International publication number: WO 2024/101853

(57) **Abstract**

The present invention provides a cancer diagnostic kit comprising a protein biomarker, Defensin-1 alpha (DEFA), which enables the rapid and simple diagnosis of cancer by quantitatively measuring the concentration of the biomarker in the serum of various cancer patients.

## Description

### TECHNICAL FIELD

The present invention relates to a cancer diagnostic kit, and more particularly to a cancer diagnostic kit comprising protein biomarkers in blood.

### BACKGROUND ART

According to data published by the National Statistics Portal in 2022, the number of total cancer cases (C00-C96) in 2019 was 254,718 (295.8 per 100,000 persons) [refer to KOSIS]. Compared to 586,990 cancer cases from 2001 to 2005, those from 2015 to 2019 increased to 1,057,524. While the 5-year relative survival rate was relatively high at 70.7% betweenn 2015 and 2019 (compared to 54.1% from 2001 to 2005), the number of deaths increased steadily, reaching 82,204 in 2020 (compared to 58,197 in 2000). Based on this, extensive research has been conducted to identify cancer targets with high diagnostic sensitivity and accuracy, using various human-derived samples such as cancer and surrounding tissues, blood, urine, feces, and saliva.

Although cancer diagnosis has traditionally relied on invasive methods such as tissue biopsy, liquid biopsy which is a non-invasive method enables easier sample collection and is used to assess cancer occurrence and metastasis. An ideal biomarker should be highly sensitive and specific for disease diagnosis, and measurable by a simple and low-risk method. Treatments should be adjusted based on the biomarker, and testing needs to be quick and accurate to allow for clinical applications.

Many research groups have actively developed cancer biomarkers based on bloods containing ctDNA, CTCs, exosomes, circulating RNA, miRNA, and TEPs that have been conventionally used to detect cancers. However, these methods often suffer from low concentrations in blood, poor detection efficiency, and lack of validated analysis methods. Furthermore, the process of separating these biomarkers from blood is complex, complicating the overall diagnosis and limiting rapid testing. Relatedly, Korean Patent No. 2012-0009903 discloses a cancer diagnostic kit and pharmaceutical composition for cancer prevention and treatment.

### DETAILED DESCRIPTION OF THE INVENTION

### TECHNICAL PROBLEM

However, the aforementioned prior art involves complex procedures for identifying and testing cancer diagnostic biomarkers, making simple diagnosis difficult. The present invention seeks to solve these and other problems by providing a cancer diagnostic kit comprising a blood protein biomarker that enables fast and simple diagnosis of cancer by quantitatively measuring the concentration of Defensin-1 alpha (DEFA) in serum from various cancer patients. The above-described problem is illustrative and does not limit the scope of the present invention.

### TECHNICAL SOLUTION

In one aspect of the present invention, there is provided a kit for diagnosing cancer comprising a reagent for detecting the Defensin-1α protein marker present in a sample obtained from a subject as an active ingredient.

In another aspect of the present invention, there is provided a method for providing information for cancer diagnosis, comprising measuring the concentration of Defensin-1α protein in a serum sample obtained from a subject.

In another aspect of the present invention, there is provided a method for diagnosing cancer, comprising measuring the concentration of Defensin-1α protein in a serum sample obtained from a subject.

In another aspect of the present invention, there is provided the Defensin-1α protein for use in cancer diagnosis.

### EFFECT OF THE INVENTION

The cancer diagnostic kit comprising blood protein biomarkers according to the present invention enables fast and simple cancer screening based on the significantly reduced concentration of Defensin-1 alpha (DEFA) in the serum of cancer patients (including liver, pancreatic, colorectal, prostate, and gastric cancer) compared to healthy individuals. Thus, it can be used for early diagnosis and prognosis monitoring of cancer. Additionally, the kit may be used in hospitals for large-scale testing and rapid on-site diagnosis. Of course, the scope of the invention is not limited by these effects.

### BRIEF DESCRIPTION OF THE DRAWINGS

FIG. 1 is a graph showing the results of analyzing the concentration of Defensin-1 alpha biomarker in the serum of healthy individuals and various cancer patients.
FIG. 2 is a graph showing the results of analyzing the concentration of Defensin-1 alpha biomarker in the serum of healthy individuals and patients with liver, pancreatic, colorectal, prostate, and gastric cancer.

### BEST MODE FOR CARRYING OUT THE INVENTION

### Definitions of terms:

As used herein, the term "Defensin-1 alpha (DEFA)" refers to a cationic antimicrobial peptide of 2-6 kDa with three pairs of intramolecular disulfide bonds. It belongs to the alpha subfamily of mammalian defensin peptides and is also referred to as cryptdin in mammals. It is produced in the small intestine and exhibits activity against many Gram-negative and Gram-positive bacteria, fungi, and enveloped viruses. It is particularly abundant in neutrophils, certain macrophage populations, and Paneth cells of the small intestine.

The term "antibody mimetic" as used herein refers to a protein that functions similarly to an antibody but is not derived from conventional full-length antibodies, which typically function as a heterotetramer composed of two heavy chains and two light chains. Instead, antibody mimetics are derived from non-antibody protein scaffolds, such as nanobodies, monobodies, and variable lymphocyte receptors (VLRs).

The term "diagnosis" as used herein includes all types of analyses used to predict the likelihood of disease occurrence, or to determine or infer the risk of disease occurrence.

The term "biomarker" as used herein refers to a substance that can be used to diagnose a disease, i.e., a substance that can differentiate between the presence or absence of cancer in a biological sample. It includes organic biomolecules such as polypeptides, nucleic acids (e.g., mRNA), lipids, glycolipids, glycoproteins, and sugars (monosaccharides, disaccharides, oligosaccharides, etc.) that show significant differences in healthy subjects and in those with cancer.

The term "early gastric cancer (EGC)" as used herein refers to a malignant tumor occurring in the stomach, where cancer cells are confined to the mucosa or submucosa, regardless of lymph node metastasis.

The term "advanced gastric cancer (AGC)" as used herein refers to a condition in which cancer has invaded into the muscularis propria of the stomach or deeper, running through the submucosal layer. The cancer is not confined to the stomach alone, but is likely to have spread to regional lymph nodes or to adjacent organs such as the liver, pancreas, transverse colon, and spleen, or to distant organs such as the liver, lungs, and bones via lymphatic or vascular pathways.

### Detailed descriptions of the invention:

According to one aspect of the present invention, there is provided a kit for diagnosing cancer, comprising a reagent for detecting the Defensin-1α protein marker present in a sample obtained from a subject as an active ingredient.

In the kit, the sample may be selected from the group consisting of blood, plasma, and serum, and the cancer may be selected from the group consisting of liver cancer, pancreatic cancer, colorectal cancer, prostate cancer, and gastric cancer. The gastric cancer may be early gastric cancer (EGC) or advanced gastric cancer (AGC).

In the kit, the detection reagent may be an antibody that specifically binds to the protein, a functional fragment of the antibody, an antibody mimetic, an aptamer, a compound, or a reagent for mass spectrometry. The functional fragment of the antibody may be Fab, F(ab')2, Fab', scFv, di-scFv, sdAb, VHH, or VNAR. The antibody mimetic may be selected from the group consisting of Affilin, Affibody, Affimer, Affitin, Alphabody, Anticlin, Avimer, DARPin, Fynomer, Kunitz domain peptide, monobody, variable lymphocyte receptor (VLR), Minibody, Diabody, Tetrabody, Triabody, and Peptabody.

The kit may have the form of enzyme-linked immunosorbent assay (ELISA) or lateral-flow immunoassay (LFA).

According to another aspect of the present invention, there is provided a method for providing information for cancer diagnosis, comprising measuring the concentration of Defensin-1α protein from a serum sample obtained from a subject.

In the method, the step of diagnosing cancer when the measured concentration of Defensin-1α protein is significantly lower than that of a normal subject can be further included. The subject may be a human or a non-human mammal.

According to another aspect of the present invention, there is provided a method of diagnosing cancer, comprising the step of measuring the concentration of Defensin-1α protein from a serum sample obtained from a subject. In this diagnostic method, a step of diagnosing cancer may be further included when the concentration of the Defensin-1α protein is significantly lower than in normal subjects.

According to another aspect of the present invention, there is provided the Defensin-1α protein used in cancer diagnosis. The protein may be used *in vitro* or *ex vivo* for cancer diagnosis.

The cancer diagnostic kit of the present invention may further comprise suitable reagents and tools for analysis, such as appropriate carriers, labels that can produce detectable signals, solvents, washing agents, etc. If the labeling substance is an enzyme, substrates capable of measuring enzyme activity and reaction-stopping agents may also be included.

Suitable carriers may include, but are not limited to, soluble carriers such as physiologically acceptable buffers known in the art (e.g., PBS), and insoluble carriers such as polymers (e.g., polystyrene, polyethylene, polypropylene, polyester, polyacrylonitrile, fluororesin, cross-linked dextran, polysaccharide, metal-plated latex magnetic microbeads), or others including paper, glass, metal, agarose, or combinations thereof.

In the case of an ELISA kit, preferably, it may include reagents capable of detecting an antibody bound to the marker protein, such as labeled secondary antibodies, chromophores, enzymes (e.g., conjugated to the antibody), and their substrates. Antibodies specific to a quantification control protein may also be included.

The antibodies may be modified by conjugation to various molecules such as enzymes, fluorescent substances, radioactive substances, and proteins. Such modified antibodies can be obtained through conventional chemical modification methods known in the art. The antibody may be a chimeric antibody or a humanized antibody, wherein the chimeric antibody is formed by combining a variable region derived from a non-human antibody with a constant region derived from a human antibody, and the humanized antibody is formed by combining a frame work region (FR) derived from a human antibody or a complementarity-determining region derived from a non-human antibody with a constant region. These antibodies may be prepared using methods known in the art. Analytical methods include, but are not limited to, Western blotting, enzyme linked immunosorbent essay (ELISA), radioimmunoassay, radioimmunodiffusion, Ouchterlony double diffusion, rocket immunoelectrophoresis, immunohistochemistry, immunoprecipitation assay, complement fixation assay, FACS, protein chip, etc.

With the proliferation of omics technologies, screening for biomarker development has become easier, leading to increased research for developing relevant diagnostic markers. Diagnostic markers are also used in the evaluation phase of new drug development processes. Currently, clinically used biomarkers include blood glucose for diagnosis of diabetes and blood cholesterol for that of hyperlipidemia. For cancer diagnosis, Prostate-Specific Antigen (PSA) for prostate cancer, Carcinoembryonic Antigen (CEA) for colorectal cancer, and Alpha-fetoprotein (AFP) for testicular and liver cancers are representative examples. Biomarkers can be categorized by their characteristics and usage into screening biomarkers, prognostic biomarkers, stratification biomarkers, efficacy biomarkers, mechanism of action biomarkers, toxicity biomarkers, translation biomarkers, disease biomarkers, staging biomarkers, surrogate biomarkers, and target biomarkers. However, although various biomarkers are being developed for different cancer types and applications, commercial product stability remains insufficient. Even within the same cancer type, the genetic characteristics of tumors vary among patients, and gene expression patterns differ at the tumor center and invasive front in a single biopsy. Additionally, tumor characteristics differ between primary and metastatic sites, making it difficult to derive highly sensitive and accurate target markers using tissue samples. In contrast, liquid biopsy such as blood better reflects the consistent tumor environment and allows convenient sample collection during routine clinical pathology procedures. Representative examples of blood-based targets used in developing cancer biomarkers include circulating tumor DNA (ctDNA), circulating tumor cells (CTCs), microvesicles and exosomes, circulating RNA and microRNAs (miRNAs), and tumor-educated platelets (TEPs). However, ctDNA and CTCs have low blood concentration and detection efficiency; microvesicles and exosomes are difficult to distinguish and pose challenges in detection; and miRNA requires further verification of analysis and data processing methods, making the diagnostic process complex and slow. Therefore, there is a need to develop cancer markers that can be applied directly to blood samples with minimal preprocessing. While no literature has been identified that presents Defensin-1 alpha (DEFA) as a blood-based cancer biomarker, some studies report its association with specific cancers and human diseases (e.g., Daniel A. Holterman et al., Urol Oncol. Mar-Apr;24(2):97-108, 2006). Accordingly, the present inventors aimed to identify serum-based target markers for cancer diagnosis, and have developed a cancer diagnostic kit containing serum-based blood protein biomarkers by verifying the diagnostic sensitivity and accuracy of target proteins by cancer types obtained after a serum analysis. By simplifying the treatment process of blood samples and applying them directly, the concentration of Defensin-1 alpha (DEFA) in serum was quantitatively measured using the kit, and it was confirmed that DEFA levels were significantly reduced in patients with liver, pancreatic, colorectal, prostate, and gastric cancers compared to healthy controls. Therefore, the kit of the present invention can be applied in ELISA or LFA formats using serum samples, enabling rapid determination of cancer presence and quick cancer screening from routine blood collection during health check-ups. Thus, the kit can be applied to early cancer treatment and prognosis monitoring.

The present invention will now be described in more detail with reference to the following examples. However, the present invention is not limited to the embodiments disclosed herein, but may be embodied in many different forms, and the following embodiments are provided to make the disclosure of the invention complete and to give those of ordinary skill in the art a complete idea of the scope of the invention.

### Example 1: Collection of Serum Samples

The present inventors collected serum samples from 249 individuals, including healthy subjects and cancer patients, as follows. A tourniquet was applied to the arm of each subject, and 5 mL of blood was drawn using a serum separating tube (SST). The samples were then gently mixed to allow clotting and subsequently centrifuged to collect the serum. The collected serum samples were stored at -80°C in an ultra-low temperature freezer until use. In total, the collected serum samples included: 50 from healthy individuals, 20 from liver cancer patients, 20 from breast cancer patients, 20 from pancreatic cancer patients, 20 from colorectal cancer patients, 20 from ovarian cancer patients, 20 from prostate cancer patients, 20 from lung cancer patients, 24 from early gastric cancer (EGC) patients, and 35 from advanced gastric cancer (AGC) patients.

### Example 2: Analysis of Diagnostic Marker Expression Levels

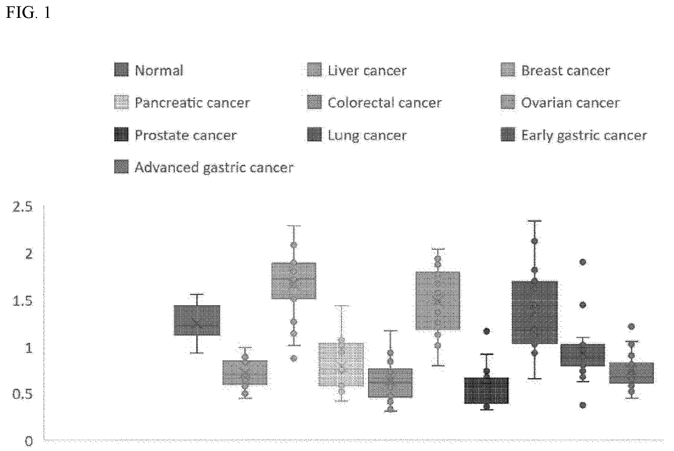

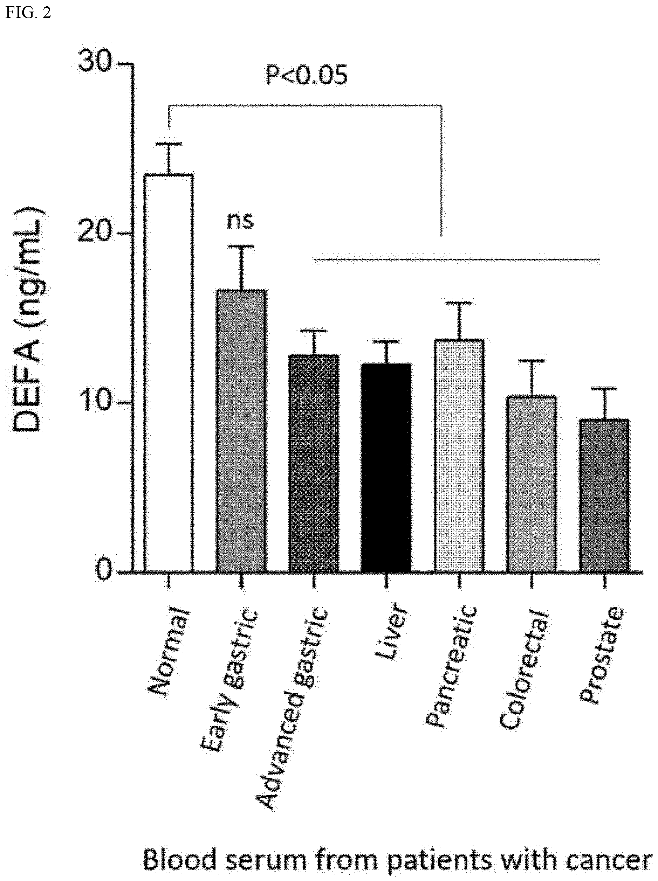

The present inventors quantitatively analyzed the level of defensin-1α protein in the collected serum samples from healthy individuals and patients with liver, pancreatic, colorectal, prostate, and gastric cancers using a Human α-defensin 1 ELISA kit (Cat no. DY8198-05, R&D Systems). As a result, it was confirmed that the concentration of human defensin-1α was significantly decreased in patients with certain types of cancer (liver, pancreatic, colorectal, prostate, and gastric cancer) compared to healthy individuals (FIG. 1). Among these cancers, the decrease was particularly notable in liver, colorectal, prostate, and gastric cancers. In the case of gastric cancer, both early gastric cancer (EGC) and advanced gastric cancer (AGC) showed significantly lower levels compared to the healthy group (FIG. 2). These results suggest that defensin-1α according to the present invention can serve as a biomarker for the rapid and accurate diagnosis of various cancers. The serum concentrations of defensin-1α quantified through the standard curve analysis are summarized in Table 1 below.

**Table 1: The concentration of human defensin-1α in serum**

| Classification | Concentration in serum | |
|---|---|---|
| | Average (ng/ml) | Standard Deviation |
| Normal | 23.76 | ± 4.11 |
| Gastric cancer (EGC) | 16.94 | ± 5.97 |
| Gastric cancer (AGC) | 12.77 | ± 3.67 |
| Liver cancer | 12.24 | ± 3.24 |
| Pancreatic cancer | 13.83 | ± 5.50 |
| Colorectal cancer | 10.49 | ± 4.71 |
| Prostate cancer | 9.06 | ± 4.37 |

The present invention has been described with reference to the embodiments described above, but these are exemplary only, and one having ordinary skill in the art will understand that various modifications and other equally valid embodiments are possible from them. The true scope of technical protection of the invention should therefore be determined by the technical ideas of the appended claims of the patent.

### (Government-funded Research and Development Project Supporting This Invention)

- **Project Unique Number:** 2016111010
- **Project Number:** NRF-2016R1D1A1B01012760
- **Ministry:** Ministry of Education
- **Project Managing (Specialized) Organization:** National Research Foundation of Korea
- **Research Project Title:** Basic Research Program in Science and Engineering
- **Research Title:** Development of On-site Diagnostic Surface-enhanced Fluorescent Signal Amplification and CMOS-based Skin-on-α-Chip Biosensor for Alternative Evaluation of Animal Testing
- **Contribution Rate:** 1/1
- **Executing Organization:** Industry-Academic Cooperation Foundation of Eulji University
- **Research Period:** June 1, 2016 - May 31, 2022

## Claims

1. A kit for diagnosing cancer comprising a reagent for detecting a Defensin-1α protein marker present in a sample obtained from a subject, as an active ingredient.

2. The kit according to claim 1, wherein the sample is selected from the group consisting of blood, plasma, and serum.

3. The kit according to claim 1, wherein the cancer is selected from the group consisting of liver cancer, pancreatic cancer, colorectal cancer, prostate cancer, or gastric cancer.

4. The kit according to claim 1, wherein the gastric cancer is early gastric cancer (EGC) or advanced gastric cancer (AGC).

5. The kit according to claim 1, wherein the detection reagent is an antibody specifically binding to the protein, a functional fragment of the antibody, an antibody mimetic, an aptamer, a compound, or a reagent for mass spectrometry.

6. The kit according to claim 5, wherein the functional fragment of the antibody is Fab, F(ab')₂, Fab', scFv, di-scFv, sdAb, VHH, or VNAR.

7. The kit according to claim 5, wherein the antibody mimetic is selected from the group consisting of Affilin, Affibody, Affimer, Affitin, Alphabody, Anticalin, Avimer, DARPin, Fynomer, Kunitz domain peptide, monobody, variable lymphocyte receptor (VLR), minibody, diabody, tetrabody, triabody, or peptabody.

8. The kit according to claim 1, wherein the detection is performed in the form of enzyme-linked immunosorbent assay (ELISA) or lateral-flow immunoassay (LFIA).

9. A method for providing information for cancer diagnosis, comprising measuring the concentration of Defensin-1α protein in a serum sample obtained from a subject.

10. The method according to claim 9, further comprising diagnosing cancer when the concentration of the Defensin-1α protein is significantly lower than that in a normal subject.

11. A method for diagnosing cancer, comprising measuring the concentration of Defensin-1α protein in a serum sample obtained from a subject.

12. A Defensin-1α protein for use in cancer diagnosis.
